# EUROPEAN PATENT APPLICATION

(11) **EP 4 190 336 A1**
(43) Date of publication of application: **07.06.2023**
(21) Application number: 21848869.0
(22) Date of filing: 30.07.2021
(51) Int. Cl.: A61K 35/28, A61P 11/00, A61P 31/14, A61P 9/00, A61P 31/04, A61P 31/10

(54) **APPLICATION OF MESENCHYMAL STEM CELLS IN PREPARATION OF DRUG FOR REPAIRING LUNG DAMAGE CAUSED BY COVID-19**

(30) Priority: 30.07.2020 CN 202010747824
(71) Applicant: Fifth Medical Center of Chinese PLA General Hospital, Beijing 100071 (CN); Vcanbio Cell & Gene Engineering Corp., Ltd, Tianjin 300450 (CN)
(72) Inventor: WANG, Fusheng, Beijing 100071 (CN); SHI, Lei, Beijing 100071 (CN); XU, Ruonan, Beijing 100071 (CN); YUAN, Xin, Beijing 100071 (CN); ZHANG, Yu, Tianjin 300450 (CN); YAO, Weiqi, Tianjin 300450 (CN)
(74) Representative: Murgitroyd & Company
(86) International application number: PCT/CN2021/109807
(87) International publication number: WO 2022/022707

(57) **Abstract**

Provided is a use of mesenchymal stem cells in the preparation of a drug for repairing lung damage caused by COVID-19. It is proved by clinical tests for the first time that mesenchymal stem cells have an excellent effect on repair of lung damage in subjects with viral pneumonia, especially severe pneumonia caused by SARS-CoV-2 infection, and can significantly reduce solid component lesion volume of the subjects, and repair lung tissue; moreover, it is proved by means of a six minute walking test that mesenchymal stem cells can significantly repair the subjects' cardiopulmonary function, so that the aerobic exercise capacity is significantly improved, thereby effectively alleviating sequelae of the subjects after the end of antiviral treatment, and improving health and quality of life of the subjects; in addition, mesenchymal stem cells are highly safe, and no obvious adverse reactions have been found.

## Description

### TECHNICAL FIELD

The present disclosure belongs to the field of biomedicine and relates to use of mesenchymal stem cells (MSCs), and in particular to use of mesenchymal stem cells in the preparation of a drug for repairing/treating lung damage.

### BACKGROUND

The main symptoms of corona virus disease 2019 (COVID-19) caused by corona virus SARS-CoV-2 include dyspnea and lung damage. COVID-19 is characterized by continuous cytokines and immune-mediated excessive inflammation, which can lead to severe respiratory diseases with a mortality rate of 2% to 5%. Severe patients with COVID-19 are susceptible to inflammatory factor storms due to the release of various inflammatory cells, which will lead to increased lung microvascular permeability and lung edema, and thus to acute lung damage. Meanwhile, due to SARS-CoV-2 virus infection, severe patients with COVID-19 are prone to pulmonary exudative lesions and develop into pulmonary fibrosis.

Human mesenchymal stem cells are heterogeneous stem cells, which exist in tissues such as bone marrow, umbilical cord, fat, and the like of mammals, and can differentiate into a variety of pluripotent stem cells such as adipocytes, chondrocytes, and osteocytes. Mesenchymal stem cells have been found to express a large number of chemokines and cytokine receptors, and are capable of combating inflammation in local tissues. Chemokines and cytokines are released during inflammation and initiate an inflammatory response. These same signal transduction molecules can facilitate MSCs to find inflammatory sites through CXCR4 receptors and the like on the surface of MSCs. Furthermore, MSCs can target dendritic cells to anti-inflammatory T cell responses via signal transduction or regulate innate immune responses by inhibiting the function of natural killer cells. MSCs also play an immunomodulatory role through direct interaction with activation of CD4+T cells to influence adaptive immune responses. Therefore, mesenchymal stem cells have been used in anti-inflammatory or immunotherapy.

### TECHNICAL PROBLEM

For severe subjects with viral pneumonia, even after the inflammatory factors are suppressed by symptomatic treatment, there are still symptoms such as impaired lung function and interstitial lung lesion for a long time, which seriously affects their quality of life.

### TECHNICAL SOLUTION

The present disclosure provides a use of a mesenchymal stem cell in the preparation of a drug for repairing/treating lung damage, wherein the lung damage comprises damage to lung in function and/or tissue.

The present disclosure provides a method for repairing/treating damage to lung in function, which comprises administering a mesenchymal stem cell to a subject with lung damage.

The present disclosure also provides a method for treating or repairing lung fibrosis, which comprises administering mesenchymal stem cells to a subject.

According to the present disclosure, the lung tissue damage includes solid component or ground-glass lesions on lung imaging radiography caused by any possible reasons.

According to the present disclosure, the damage to lung in function includes at least one of the following symptoms: dyspnea (RR≥30 beats per minute), finger blood oxygen saturation at rest ≤ 93%, and arterial oxygen partial pressure (PaO₂)/oxygen absorption concentration (FiO₂) ≤ 300 mmHg.

### TECHNICAL EFFECTS

Inventors of the present disclosure have found that mesenchymal stem cells can be used for the treatment of lung damage, especially including damage to lung in function and tissue, which can be caused by viral infection. In the present disclosure, it is proved by clinical tests for the first time that mesenchymal stem cells have an excellent effect on the repair/treatment of lung damage in subjects with viral pneumonia, especially severe pneumonia caused by SARS-CoV-2 infection, and can significantly reduce solid component lesion volume of the subjects, and repair lung tissue. Moreover, it is proved by means of a six-minute walking test that mesenchymal stem cells can significantly repair/recover the subjects' cardiopulmonary function, so that the aerobic exercise capacity is significantly improved, thereby effectively alleviating sequelae of the subjects after the end of antiviral treatment, and improving health and quality of life of the subjects; in addition, mesenchymal stem cells are of high safety, and no obvious adverse reactions have been found.

### BRIEF DESCRIPTION OF THE DRAWINGS

The technical solutions and other beneficial effects of the present disclosure will be clear by describing the specific embodiments of the present disclosure in detail with the attached drawings.
FIG. 1 shows a histogram of data distribution of a six-minute walking test of the subjects;
FIG. 2 shows a box-plot of data distribution of a six-minute walking test of the subjects, wherein unit of the ordinate is meter;
FIG. 3 shows a histogram of data distribution of relative change values in total lesion volume percentage of whole lung volume in subjects;
FIG. 4 shows a box-plot of data distribution of relative change values in total lesion volume percentage of whole lung volume in subjects;
FIG. 5 shows a histogram of data distribution of relative change values in total solid component lesion volume percentage of whole lung volume;
FIG. 6 shows a box-plot of data distribution of relative change values in total solid component lesion volume percentage of whole lung volume;
FIG. 7 shows a histogram of data distribution of relative change values in total ground-glass lesion volume percentage of whole lung volume;
FIG. 8 shows a box-plot of data distribution of relative change values in total ground-glass lesion volume percentage of whole lung volume;

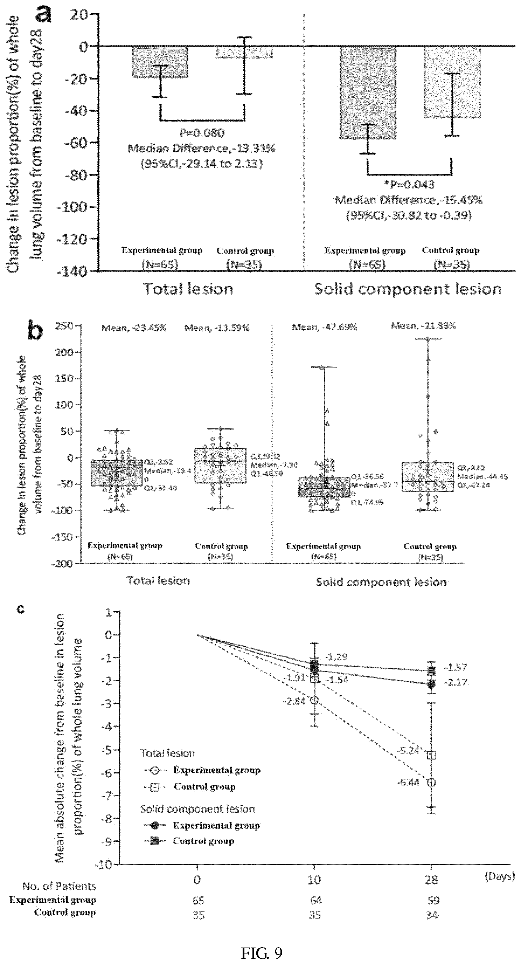

FIG. 9 shows effects after repairing/treating lung damage in patients with severe COVID-19 by using human umbilical cord mesenchymal stem cells (UC-MSCs); a shows the median difference between groups in total lesion proportion (%) of whole lung volume and solid component lesion proportion (%) of whole lung volume from baseline to day 28; b shows a box-plot of total lesion proportion (%) of whole lung volume and solid component lesion proportion (%) of whole lung volume from baseline to day 28, wherein Q1 represents a first quartile, Q3 represents a third quartile, and I bar represents the minimum and maximum values; c shows the mean absolute change in total lesion proportion (%) of whole lung volume and solid component lesion proportion (%) of whole lung volume from baseline to day 28, wherein I bar represents the standard error.

### DETAILED DESCRIPTION

Hereinafter, technical solutions in embodiments of the present disclosure will be clearly and completely described with reference to the accompanying drawings in embodiments of the present disclosure. Apparently, the described embodiments are part of, not all of, the embodiments of the present disclosure. All the other embodiments, obtained by a person with ordinary skill in the art on the basis of the embodiments in the present disclosure without expenditure of creative effort, belong to the protection scope of the present disclosure.

According to the present disclosure, the mesenchymal stem cells are particularly useful for lung damage caused by a respiratory infection in subjects. The respiratory infection comprises viral pneumonia, bacterial pneumonia or fungal infection. In an embodiment, the viral pneumonia is a severe or critical type of pneumonia caused by any one or more of coronavirus SARS-CoV-2, SARS-Cov or MERS-Cov. In a preferred embodiment, the mesenchymal stem cells especially have a good effect on repairing/treating lung damage caused by corona virus SARS-CoV-2 infection.

According to the present disclosure, the mesenchymal stem cells are pluripotent stem cells (i.e., stem cells capable of differentiating into daughter cell types), which are capable of differentiating intovarious cell types. The mesenchymal stem cells may be derived from a variety of tissues, including but not limited to, human tissues such as bone marrow tissue, adipose tissue, muscle tissue, reproductive tissue (e.g., menstrual blood, amniotic membrane, amniotic fluid, or umbilical cord tissue), skin tissue, bone tissue, and dental tissue. The mesenchymal stem cells may also be isolated or induced from various other types of human cells, including but not limited to, germ cells, embryonic cells, adult cells, and the like. The mesenchymal stem cells may be obtained by using techniques known in the art such as isolation, purification, culture and amplification. The mesenchymal stem cells may be derived from a variety of sources, including autologous, allogenic or xenogenic sources.

According to the present disclosure, the mesenchymal stem cells may be homogeneous compositions or may be mixed cell clusters comprising the mesenchymal stem cells. Suitable MSCs are available from umbilical cord tissue. In some embodiments, the mesenchymal stem cell compositions are obtained by adherently culturing the umbilical cord wharton's jelly in a suitable medium. MSCs can be identified by specific cell surface markers.

According to the present disclosure, the drug for repairing/treating lung damage is in a form of injection formulation and can be administered to a subject through a variety of modes of administration. In some embodiments, the mesenchymal stem cells are administered systemically, for example, by intravenous, intra-arterial or local administration.

The dose of mesenchymal stem cells to be administered depends on many factors, including the age, weight and sex of the subject and the severity of lung damage. Practitioners with qualified level in this field have the ability to determine their therapeutically effective amount according to the subject's situation, for example, in an amount of 1×10⁵ cells /kg body weight to 1×10⁸ cells /kg body weight.

According to the present disclosure, the drug for repairing/treating lung damage comprises mesenchymal stem cells and a pharmaceutically acceptable drug carrier. For example, the mesenchymal stem cells are in the form of a cell suspension dispersed in an acceptable liquid dispersant or gel for intravenous injection or topical administration. In an embodiment, the liquid dispersant is a physiological saline solution, and the drug for repairing/treating lung damage is a physiological saline suspension of mesenchymal stem cells. The physiological saline suspension of mesenchymal stem cells may contain other physiologically acceptable components such as dimethyl sulfoxide (DMSO) and/or human serum albumin.

In an embodiment, the physiological saline suspension of mesenchymal stem cells has a cell concentration of 0.5×10⁵ cells/mL to 5.0×10⁵ cells/mL, preferably 4.0×10⁵ cells/mL.

According to the present disclosure, the drug for repairing/treating lung damage contains more than 95%, preferably more than about 98% by mass of the mesenchymal stem cells.

As used herein, the terms "subject(s)" and "patient(s)" interchangeably refer to living organisms suffering from or prone to diseases that can be treated by administering the pharmaceutical composition described herein, and non-limiting examples include humans, non-human primates or other mammals. For example, subjects of the present disclosure include adults, infants, children, and other warm-blooded mammals including, but not limited to, non-human primates such as chimpanzees, other apes or monkeys, and other zoo animals, domestic mammals or laboratory animals, such as cats, pigs, dogs, cattle, sheep, mice, rats, guinea pigs, and the like. Preferably, the "subject" in the present disclosure is human being.

As used herein, "repair/treat" or "reparing/treating" refers to preventing or arresting deterioration of an damage through medical actions carried out on the subject when a functional damage to a tissue or organ has been caused and the state of which is lower than an average level of a normal individual, and reducing or alleviating the degree of damage as much as possible, so as to make the damage turned into a healthy state. In an embodiment, repairing lung damage includes the reduction of lung lesion tissues and/or the improvement of cardiopulmonary function, including but not limited to the relief of dyspnea symptoms, the increase of oxygenation index, the reduction of solid component lesion volume, and the enhancement of aerobic exercise ability at rest and/or exercise. In the embodiments of the present disclosure, the effect of repairing lung tissue is mainly evaluated by solid component lesion volume obtained from lung imaging examination, and the effect of improving lung respiratory function is evaluated by a 6-minute walking distance.

The severe or critical type of pneumonia described herein is determined according to the diagnosis and treatment criteria for pneumonia classification known in the art. In some embodiments, an adult patient who meets any of the following conditions belongs to a severe patient: (1) shortness of breath, RR ≥ 30 beats per minute; (2) finger blood oxygen saturation at rest ≤ 93%; (3) arterial oxygen partial pressure (PaO₂)/oxygen absorption concentration (FiO₂) ≤ 300 mmHg; or (4) lung imaging showing a lesion progression of more than 50% within 24 hours to 48 hours. Children with any of the following symptoms are severe patients: (1) shortness of breath ( RR ≥ 60 beats per minute for children aged less than 2 months; RR ≥ 50 beats per minute for children aged 2 months to 12 months; RR ≥ 40 beats per minute for children aged 1 to 5 years old,; and RR ≥ 30 beats per minute for children over 5 years old), except for those caused by the influence of fever and crying; (2) finger blood oxygen saturation at rest ≤ 92%; (3) auxiliary breathing (groaning, flaring of alae nasi, three concave sign), cyanosis, intermittent apnea; (4) drowsiness, convulsions; or (5) apocleisis or feeding difficulties with signs of dehydration. In some embodiments, those who meet one of the following conditions belong to critical patients: (1) respiratory failure occurs and mechanical ventilation is needed; (2) shock; or (3) ICU monitoring is needed and associated with failure of other organs.

The present disclosure will be further illustrated in connection with specific embodiments, but the present disclosure is not limited to the following embodiments. Such methods are conventional methods unless otherwise specified. All the materials mentioned above can be obtained from public sources unless otherwise specified.

Experimental schemes:
This experiment was carried out after repeated demonstration, ethical review, and normative registration (NCT04288102). Subjects were followed up after discharge. This experiment is a randomized double-blind clinical trial including a placebo control group. Written informed consent was obtained from the subjects or subjects' agent.

Subjects: severe or critical types of patients with corona virus disease COVID-19, and the degree of lung fibrosis was evaluated by lung imaging.

The subject were diagnosed with COVID-19 virus nucleic acid by nasopharyngeal swab RT-PCR, combined with serum specific IgM antibody, IgG antibody detection, and lung CT imaging.

Subject's symptoms and physiological indicators meet one of the following conditions: 1) dyspnea (RR≥30 beats per minute), 2) finger blood oxygen saturation at rest ≤ 93%, 3) arterial oxygen partial pressure (PaO₂)/oxygen absorption concentration (FiO₂) ≤ 300 mmHg, and 4) lung imaging showing a lesion progression of more than 50% within 24 hours to 48 hours, and chest X-ray tomography demonstrating pneumonia and interstitial lung damage.

The basic information and baseline characteristics of the subjects in the experimental group and the control group are shown in table 1.

**Table 1 Baseline characteristics of the subjects in the experimental group and the control group**

| | | Experimental group (n=65) | Control group (n=35) |
|---|---|---|---|
| Age, years-mean (SE) | | 60.72(9.14) | 59.94(7.79) |
| Sex - no. (%) | | | |
| | Man | 37(56.92%) | 19(54.29%) |
| | Woman | 28(43.08%) | 16(45.71%) |
| Weight (kg) | | 66.02(9.44) | 67.50(9.51) |
| From symptoms to treatment in this study, (unit: days) | | 43.32(10.66)/45.00 (39.00, 51.00) | 46.54(8.81)/47.00 (41.00,53.00) |
| Medical history | | 34(52.31%) | 18(51.43%) |
| Hypertension | | 17(26.15%) | 10(28.57%) |
| Diabetes | | 12(18.46%) | 5(14.29%) |
| White blood cell count (10⁹/L) | | 6.30(1.75)/ 5.70 (5.00, 6.60) | 6.25(1.89)/5.80 (5.00, 6.80) |
| Lymphocyte count (10⁹/L) | | 1.47(0.47)/ 1.39 (1.19, 1.80) | 1.60(0.62)/ 1.47 (1.24, 1.84) |
| Neutrophil count (10⁹/L) | | 3.76(1.45) / 3.48 (2.91, 4.32) | 3.93(1.44)/ 3.83 (2.85, 4.48) |
| Platelet count (10⁹/L) | | 227.57(83.03)/ 214.00(174.00, 255.00) | 217.11(59.00)/ 210.00(176.00, 247.00) |
| Hemoglobin (g/L) | | 122.68(14.44) | 124.26(11.83) |
| D-dimer (mg/L) | | 0.58(0.36,1.11) | 0.56(0.31,1.12) |
| IL-6* (pg/mL) | | 11.28(13.29)/ 7.86 (5.63, 9.84) | 14.21(23.81)/ 8.76 (6.54, 11.77) |
| CRP* (mg/L) | | 3.29(4.91) / 1.95 (0.84, 3.53) | 2.53(3.89) / 1.38 (0.68, 2.26) |

| | | | |
|---|---|---|---|
| The data in table 1 are mean (standard deviation) and median (quartile), and percentage (%). | | | |

Mesenchymal stem cells are derived from Vcanbio Cell & Gene Engineering Corp., Ltd. MSCs were obtained from the umbilical cords of full-term newborns with the consent of their guardians (UC-MSC). The umbilical cords were soaked in 75% ethanol for 10 seconds, then rinsed off with phosphate buffered saline (PBS) to remove the blood vessels, and immersed and washed with PBS for 5 minutes, and repeated for three times. The isolation and culture of UC-MSCs were carried out in a laminar flow hood under aseptic conditions. The umbilical cords were cut into small pieces of 1-2 cm to expose the umbilical cord tissue. Wharton's jelly (WJ) tissue was then cut into pieces as small as 2 mm³ from the umbilical cord tissue, and placed in an inverted tissue culture bottle (75 flaskcm²), which was cultured in 4 mL DMEM/F12 medium supplemented with fetal bovine serum (10% FBS, BI, Israel), and allowed to stand for 4 hours under the condition of 37 °C and 5% CO₂. The flask was then inverted back to the correct orientation and 10 mL of supplemental medium was added. Complete medium replacement was performed every 4 days to 5 days until the degree of fusion reached 60% to 80%. Tissue explants were removed after 8 days to 10 days of culture. Adherent cells were isolated with 1×TrypLE (GIBCO, USA), and then re-plated at a density of about 6×10³ cells per cm² to 8×10³ cells per cm² for further amplification. The master cell bank and the working cell bank were set to the highest level and a homogeneous population of these cultured cells at passage 5 (P5) was used for all experiments in this study. The harvested cells were characterized according to the minimum standards recommended by ISCT13 and showed fibroblast-like morphology with membrane-forming ability, and cell surface markers such as CD19, CD34, CD11b, CD45, CD73, CD105, CD90, HLA-DR were analyzed by flow cytometry (BD, FACS Calibur, USA) according to previous studies.

The UC-MSC product is almost colorless suspension containing 4.0×10⁷ MSCs per bag in a volume of 100 ml/bag. The placebo has the same appearance on the package.

### Dosing Regimen:

The experimental group was dosed with a therapeutic dose of 4.0×10⁷ (1 bag) per time, and each subject was dosed three times on day 0, day 3 and day 6 after random allocation. The control group was given the same dose of placebo. The total amount of UC-MSC or placebo infusion for each subject was 100 ml, and the infusion was started from a standard hemofiltration tube with an aperture of 170 µm. Under the monitoring of electrocardiogram, the drug was injected intravenously under the action of gravity for a total injection time of more than 60 minutes.

The subjects underwent chest CT examination on day 10 and day 28 after enrollment.

Six-minute walking test (6 MWT) is mainly used to evaluate the efficacy of therapeutic intervention in patients with moderate to severe cardiopulmonary diseases, and to measure the functional status of patients. It can be used as one of the key observation indicators in clinical trials and also as one of the predictors of patient survival. In this study, there was of no statistical significance in cardiac function between the treatment group and the control group, and therefore 6 MWT was used in this study as an indicator to evaluate the difference in lung function between the two groups.

The percentage of lesion volume to whole lung volume is used to evaluate lung tissue repair. The total lesion volume includes solid component lesion and ground-glass lesion, the quantification of which is determined by lung imaging examination. In this embodiment, lung imaging data of lung lesion are determined by lung CT and quantified by lung imaging software and centralized imaging interpretation analyzed by radiologists. The imaging data come from software-assisted lung volumetry and optical densitometry.

### Example 1

Embodiment 1 of the present disclosure includes an experimental group (umbilical cord mesenchymal stem cell (UC-MSC) group) including 30 subjects and a control group (placebo group) including 30 subjects randomly selected from table 1, the baseline characteristics of which are within the range of table 1, and the baseline characteristics of both groups of subjects are comparable.

For the primary outcome analysis - change in lesion proportion (%) from baseline (day 10) to day 28. T-test was used to test the difference between UC-MSCs and placebo if the assumption of normality [CZ 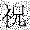 1] was satisfied, otherwise, the Mann-Whitney test was used.

### 1. Normal distribution test and homogeneity of variance test

Normal distribution test and variance homogeneity test were carried out on the experimental results, and the results of the tests are shown in table 2 and table 3 respectively.

As can be seen from tables 2 and 3, the data of 6-minute walking distance in the experimental group does not conform to the normal distribution (p < 0.05), and the data distribution of the relative change values in total solid component lesion volume percentage of whole lung volume does not conform to the normal distribution (p < 0.05). Therefore, Wilcoxon rank sum test will be used when comparing the differences between groups in the six-minute walking distance and the relative change values in total solid component lesion volume percentage of whole lung volume. T-test will be used when comparing the differences between groups in relative change values of total ground-glass lesion volume in percentage of whole lung volume.

**Table 2**

| Normal distribution test [CZ 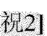 2] | | | | | | | |
|---|---|---|---|---|---|---|---|
| | Treatment group | Kolmogorov-Smimovtest * | | | Shapiro-Wilk | | |
| | | Statisti cs | Degree of freedom | Signifi cance | Stati sties | Degree of freedom | Signifi cance |
| Six-minute walking distance | Control group | ₐ165 | 13 | ₐ200^{∗} | ₐ949 | 13 | ₐ569 |
| | Experimental group | ₐ195 | 29 | ₐ006 | ₐ735 | 29 | ₐ000 |
| Relative change values in total solid component lesion volume percentage of whole lung volume | Control group | ₐ192 | 13 | ₐ200^{∗} | ₐ960 | 13 | ₐ747 |
| | Experimental group | ₐ098 | 29 | ₐ200^{∗} | ₐ968 | 29 | ₐ500 |
| Relative change values in total solid component lesion volume percentage of whole lung volume | Control group | ₐ235 | 13 | ₐ048 | ₐ823 | 13 | ₐ013 |
| | Experimental group | ₐ126 | 29 | ₐ200^{∗} | ₐ942 | 29 | ₐ113 |
| Relative change values in total ground-glass lesion volume percentage of whole lung volume | Control group | ₐ166 | 13 | ₐ200^{∗} | ₐ935 | 13 | ₐ400 |
| | Experimental group | ₐ119 | 29 | ₐ200^{∗} | ₐ955 | 29 | ₐ245 |

| | | | | | | | |
|---|---|---|---|---|---|---|---|
| *. This is a lower bound of true significance _{a.} Riley's significance correction | | | | | | | |

**Table 3 Variance homogeneity test**

| | | Levin statistics | Degree of freedom 1 | Degree of freedom 2 | Significance |
|---|---|---|---|---|---|
| Six-minute walking distance | Based on mean | 1.686 | 1 | 40 | ₐ202 |
| | Based on median | 1.020 | 1 | 40 | ₐ318 |
| | Based on the median with adjusted degrees of freedom | 1.020 | 1 | 30.162 | ₐ320 |
| | Based on trimmed mean | 1.248 | 1 | 40 | ₐ271 |
| Relative change values of | Based on mean | 2.890 | 1 | 40 | ₐ097 |
| percentage of total solid component lesion volume to whole lung volume | Based on median | 2.500 | 1 | 40 | ₐ122 |
| | Based on the median with adjusted degrees of freedom | 2.500 | 1 | 39.797 | ₐ122 |
| | Based on trimmed mean | 2.926 | 1 | 40 | ₐ095 |
| Relative change values of percentage of total solid component lesion volume to whole lung volume | Based on mean | 33.275 | 1 | 40 | ₐ000 |
| | Based on median | 14.660 | 1 | 40 | ₐ000 |
| | Based on the median with adjusted degrees of freedom | 14.660 | 1 | 12.548 | ₐ002 |
| | Based on trimmed mean | 29.918 | 1 | 40 | ₐ000 |
| Relative change values of percentage of total ground-glass lesion volume to whole lung volume | Based on mean | 2.602 | 1 | 40 | ₐ115 |
| | Based on median | 2.788 | 1 | 40 | ₐ103 |
| | Based on the median with adjusted degrees of freedom | 2.788 | 1 | 39.496 | ₐ103 |
| | Based on trimmed mean | 2.729 | 1 | 40 | ₐ106 |

### 2. Matching analysis between experimental group and control group

There were of no statistical significance (p>0.05) between the experimental group and the control group in terms of age, maximum body temperature, days from onset to admission, sex, history of heart disease, history of hypertension, history of diabetes, history of chronic obstructive lung disease, fever, fatigue, dry cough, and other symptoms (P > 0.05), and the baseline data of the two groups were comparable (as shown in table 1).

The comparison between the experimental group and the control group in terms of important indicators such as six-minute walking distance and total lesion volume is shown in table 4.

**Table 4 Indicators of six-minute walking distance and total lesion volume of the experimental group and the control group**

| Indicators | Experimental group N=30 | Control group N=15 | Statistical method | Statistics | P value |
|---|---|---|---|---|---|
| Six-minute walking distance | | | Wilcoxon rank sum test | -0.191 | 0.849 |
| N | 29 | 13 | | | |
| Mean (SD) | 411.68 (97.36) | 424.46 (42.15) | | | |
| Median | 434.00 | 432.00 | | | |
| Min to Max | 0 to 535.00 | 339.0 to 486.00 | | | |
| Relative change values in total solid component lesion volume percentage of whole lung volume | | | T test | -3.040 | 0.004 |
| N | 30 | 15 | | | |
| Mean (SD) | -33.14 (39.48) | 1.72 (28.46) | | | |
| Median | -29.17 | 1.20 | | | |
| Min to Max | -99.44 to 48.89 | -58.03 to 54.21 | | | |
| Relative change values in total solid component lesion volume percentage of whole lung volume | | | Wilcoxon rank sum test | -5.417 | <0.001 |
| N | 30 | 15 | | | |
| Mean (SD) | -76.59 (12.62) | 29.30 (83.15) | | | |
| Median | -75.06 | -9.23 | | | |
| Min to Max | -100 to 57.71 | -42.16 to 224.09 | | | |
| Relative change values in total ground-glass lesion volume percentage of whole lung volume | | | T test | -1.451 | 0.154 |
| N | 30 | 15 | | | |
| Mean (SD) | -18.08 (56.11) | 5.22 (37.34) | | | |
| Median | -18.72 | -1.38 | | | |
| Min-Max | -99.44 to 117.32 | -58.77 to 100.72 | | | |

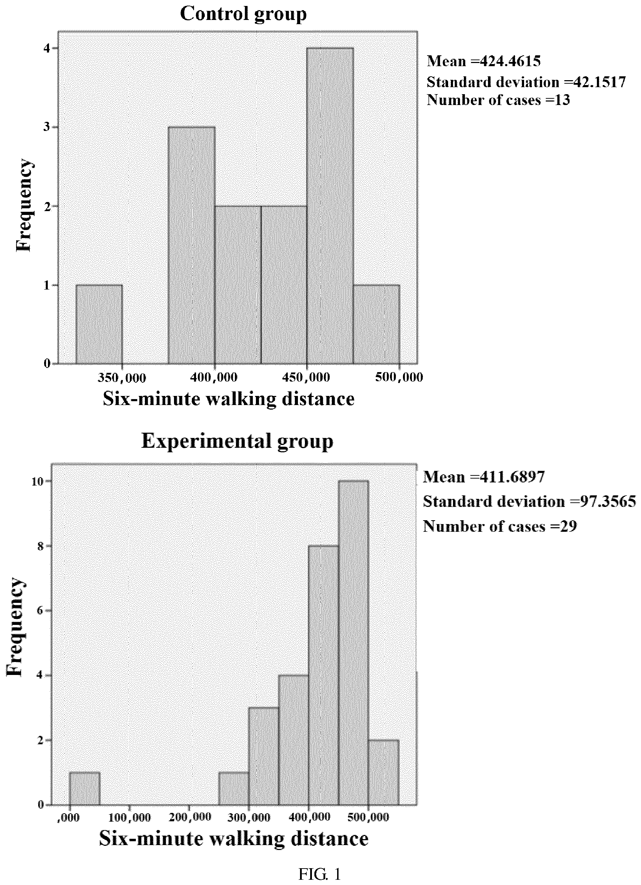

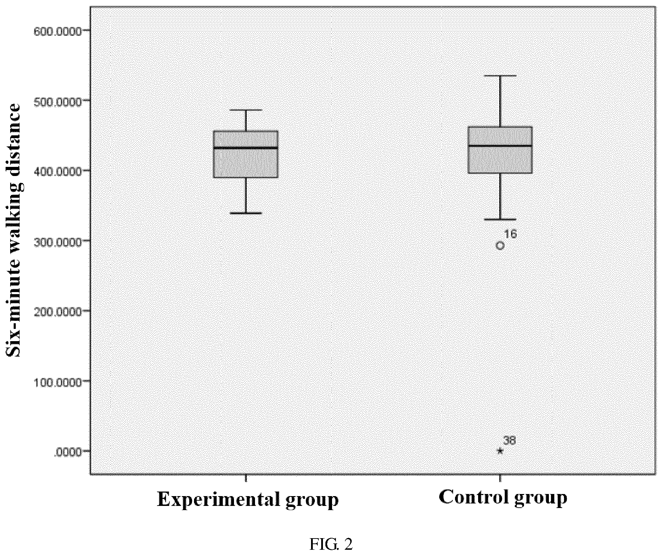

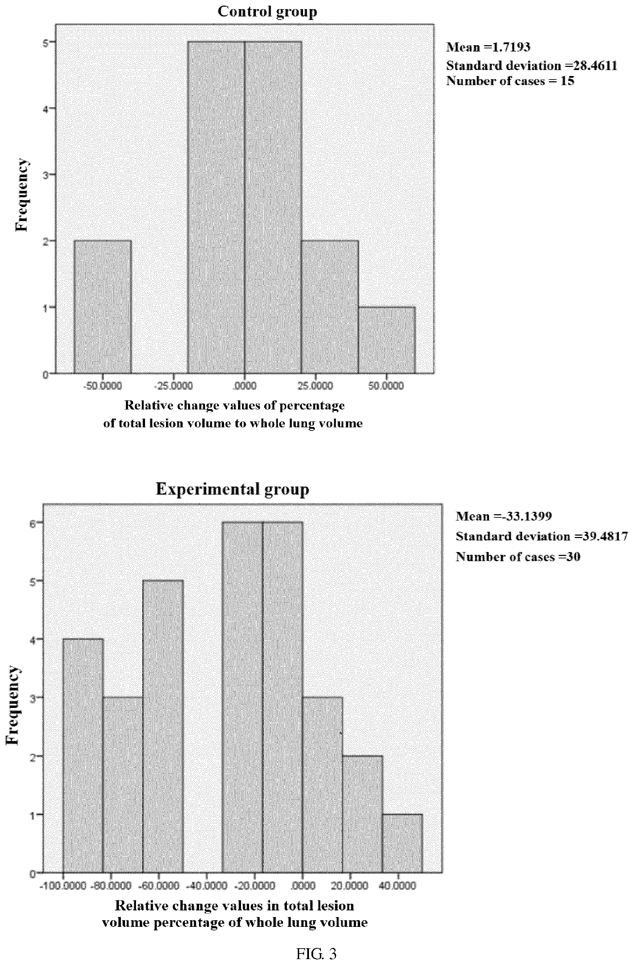

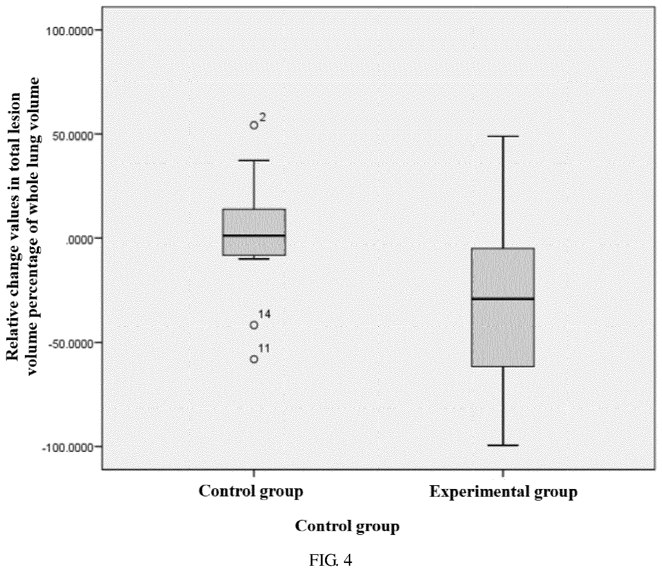

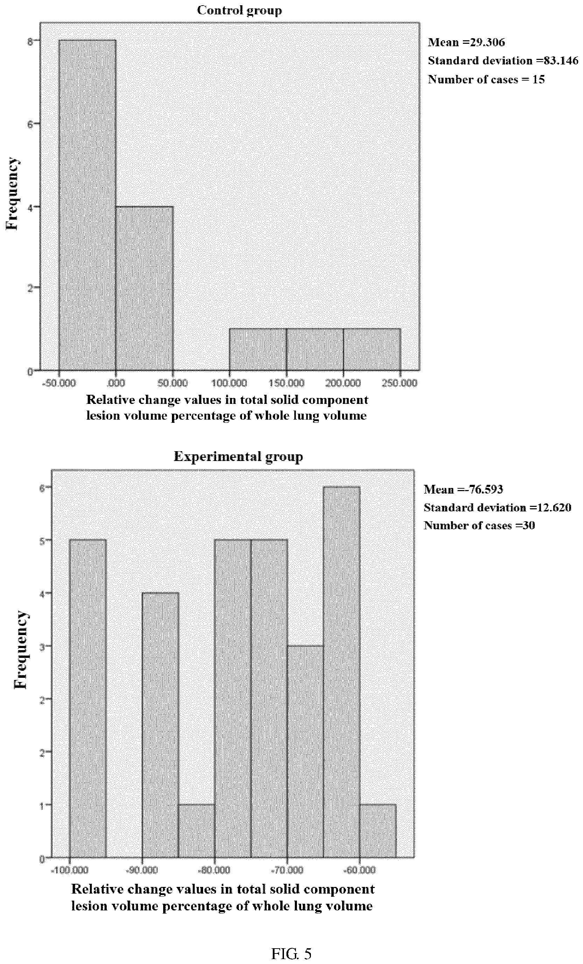

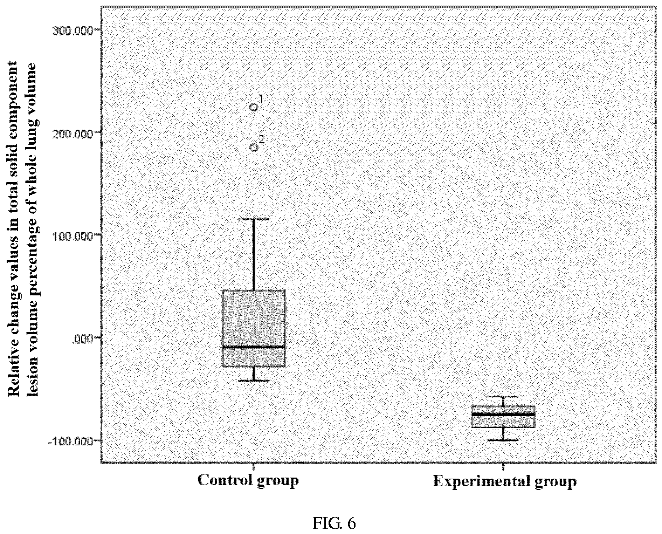

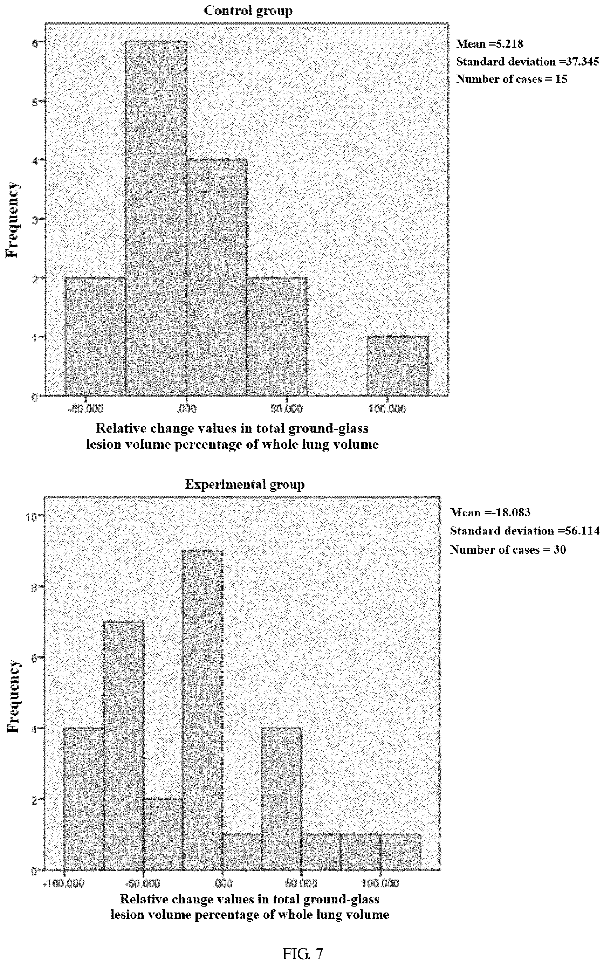

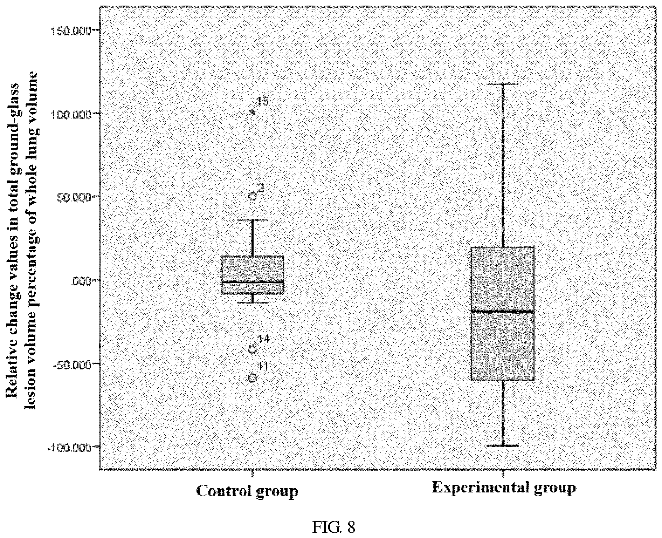

It can be seen by referring to table 4 with FIGs. 3 and 4 that in terms of the relative change values in total lesion volume percentage of whole lung volume, the recovery degree of the experimental group is better than that of the control group, and the result is statistically significant (-33.14 vs 1.72, p = 0.004 < 0.05). As can be seen from FIGs. 5 and 6, in terms of the relative change values in total solid component lesion volume percentage of whole lung volume, the recovery degree of the experimental group is better than that of the control group, and the result is statistically significant (-76.59 vs. 29.30, p < 0.001). FIGs. 7 and 8 show changes of ground-glass lesion in patients in the experimental group and the control group. Compared with placebo (ITT), changes in lung lesion proportion (%) in subjects receiving UC-MSCs from baseline to day 10 to day 28 is numerically improved. As can be seen in FIGs. 3 to 8, this experiment found that UC-MSC can significantly improve changes in solid component lesion proportion (%) of whole lung volume in severe patients with interstitial lung lesion from baseline to day 28. Interstitial lung damage (lung fibrosis) is composed of hundreds of non-neoplastic lung diseases whose clinical manifestations, morphology and development trends are different. Interstitial lung damage is a common manifestation of severe COVID-19 patients, and the interstitial lung damage caused by COVID-19 in severe patients will seriously affect the quality of life of a subject after recovery. The residual lesion in the MSCs group were significantly lower than those in the placebo group, reflecting that MSCs had potential lung fibrosis and could play a good role in repairing/treating interstitial lung damage. Although there is no statistical significance in six-minute walking distance (unit: meter) between the experimental group and the control group, it can be seen from FIGs. 1 and 2 that subjects in the experimental group are obviously superior to the control group in terms of the six-minute walking distance, and their lung function has been well recovered.

### Example 2

In order to further prove the effectiveness of mesenchymal stem cells in the repair and treatment of lung fibrosis, statistical analysis of the modified-ITT (modified intention-to treat, mITT) analysis population was performed in this embodiment. MITT was defined as 65 subjects in the experimental group and 35 subjects in the control group, of which 49 subjects in the experimental group and 25 subjects in the control group actually met the completion protocol (PP)[CZ 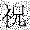 3].

All patients were treated with the above-mentioned therapeutic schedule. In order to evaluate the difference between the primary end point and the secondary end point in this clinical experiment, we analyzed and evaluated and changes in high-resolution CT images based on radiologist evaluation and lung imaging intelligent software, thus measuring lesions. The total percentage of total lesion volume in whole lung volume and total percentage of solid component lesion in whole lung volume were estimated by Hodges-Lehmann Estimation. The results were shown in FIG. 9 and table 5.

**Table 5 Primary and secondary results of mITT population**

| | Experimental group (n=65) | Control group (n=35) | Difference |
|---|---|---|---|
| Change in total lesion proportion of whole lung volume from baseline to day 28 (%) | -19.40 (-53.40, -2.62) | -7.30 (-46.59, 19.12) | -13.31 (-29.14, 2.13) |
| Change in solid component lesion proportion of whole lung volume from baseline to day 28 (%) | -57.70(-74.95, 36.56) | -44.45(-62.24, -8.82) | -15.45 (-30.82, -0.39) |
| Change in ground-glass lesion proportion of whole lung volume from baseline to day 28 (%) | -14.95 (-51.55, 7.29) | -3.94 (-43.99, 32.55) | -9.84 (-30.51, 6.86) |
| Six-minute walking distance (m) on day 28 | 420.00(392.00, 465.00) | 403.00(352.00, 447.00) | 27.00 (0.00, 57.00) |
| VCmax (L) on day 28 | 2.57 (2.13, 3.04) | 2.49 (2.05, 2.76) | 0.16 (-0.10, 0.43) |
| DLco (L) on day 28 | 5.12 (1.62) | 5.06 (1.57) | 0.07 (-0.69, 0.82) |
| *Six classes of scales on day 10* | | | 0.77 (0.33, 1.79) |
| 1-non-hospitalized | 11 (16.92%) | 6 (17.14%) | |
| 2-hospitalized without need to supplement oxygen | 8 (12.31%) | 6 (17.14%) | |
| 3-hospitalized needing to supplement oxygen | 44 (67.69%) | 23 (65.71%) | |
| 4-hospitalized using non-invasive ventilation or high flow oxygen equipment | 2 (3.08%) | 0 (0.00%) | |
| Oxygen therapy duration | 22.00 (13.00, 32.00) | 31.00 (16.00, 36.00) | -7.00 (-17.00, 3.00) |
| Finger pulse oximetry at rest on day 28 | 97.10 (1.31) | 96.97 (1.29) | 0.13 (-0.42, 0.68) |
| Scores of mMRC dyspnea on day 28 | | | 1.49 (0.68, 3.26) |
| Grade 0, n (%) | 29 (47.54%) | 13 (37.14%) | |
| Grade 1, n (%) | 24 (39.34%) | 16 (45.71%) | |
| Grade 2, n (%) | 5 (8.20%) | 4 (11.43%) | |
| Grade 3, n (%) | 3 (4.92%) | 1 (2.86%) | |
| Grade 4, n (%) | 0 (0.00%) | 1 (2.86%) | |

| | | | |
|---|---|---|---|
| Data included in table 5 are mean (standard deviation), median (quartile) or percentage (%); VCmax (L) on day 28 was derived from 53 experimental groups and 31 control groups; DLco (L) on day 28 was derived from 53 experimental groups and 27 control groups; Data of oxygen therapy duration were obtained from 29 experimental groups and 11 control groups; Data of finger pulse oximetry at rest on day 28 were obtained from 61 experimental groups and 35 control groups; and Scores of mMRC dyspnea on day 28 were derived from 61 experimental groups and 35 control groups. | | | |

The results in table 5 and FIG. 9 show that from baseline to day 28, the median difference in total lesion percentage of whole lung volume was -19.40% (95% CI, -53.40%, -2.62%) in the experimental groups, while that in the control group was -7.30% (95% CI, -46.59%, 19.12%) in the control group, with a difference of -13.31% (95% CI, -29.14%, 2.13%, P = 0.080). Taking total solid component lesion percentage of whole lung volume as the object, the median of the experimental group was -57.70% (95% CI, -74.95%, -36.56%), while that of the control group was -44.45% (95% CI, -62.24%, -8.82%), with a difference of -15.45% (95% CI, -30.82%, -0.39%, P = 0.043). A reduction in ground-glass lesion was also observed in the experimental group. It can be seen that the lung damage and lesion volume of the experimental group are significantly reduced, which has significant effect compared with the control group. It can be seen that the six-minute walking distance on day 28 is longer in the experimental group, indicating that the degree of recovery of lung function in the experimental group is better than that in the control group.

Moreover, during the experiment, the incidence of adverse events was 55.38% in the experimental group and 60% in the control group, which was generally comparable. The most common adverse events in the experimental group included an increase in the incidence of lactate dehydrogenase, which was 13.85%, while that in the control group was 20%; an increase in the incidence of serum alanine aminotransferase in the experimental group, which was 10.77%, while that in the control group was 11.43%; an increase in the incidence of hypopotassaemia in the experimental group, which was 9.23%, while that in the control group was 2.86%; an increase in the incidence of aspartate aminotransferase in the experimental group, which was 7.69%, while that in the control group was 11.43%; and the incidence of hyperuricemia in the experimental group was 7.69%, while that in the control group was 8.75%. Only one patient in the experimental group experienced a grade 3 adverse event (pneumothorax) and recovered under conservative treatment. Adverse events during the observation period were not associated with the administration of mesenchymal stem cells. There were no deaths in this experiment. Therefore, the scheme of repairing lung damage with mesenchymal stem cells provided by the present disclosure has good safety.

In summary, the present disclosure provides use of mesenchymal stem cells in repairing lung damage, which can significantly repair lung damage caused by corona virus, and has good safety.

The embodiments of the present disclosure have been described above. However, the present disclosure is not limited to the above-described embodiments. Any modifications, equivalent substitutions, modifications, etc. which fall within the spirit and principles of the present disclosure are intended to be included within the scope of protection of the present disclosure.

## Claims

1. Use of mesenchymal stem cells in the preparation of a drug for repairing lung damage, wherein the lung damage comprises damage to lung in function and/or tissue.

2. The use according to claim 1, wherein the mesenchymal stem cells is useful for a subject who suffers from lung damage caused by a respiratory infection.

3. The use according to claim 1, wherein the lung damage comprises lung fibrosis.

4. The use according to claim 3, wherein the respiratory infection comprises viral pneumonia, bacterial pneumonia or fungal infection.

5. The use according to claim 4, wherein the viral pneumonia is a severe or critical type of pneumonia caused by any one or more of coronavirus SARS-CoV-2, SARS-Cov or MERS-Cov.

6. The use according to claim 1 or 5, wherein the mesenchymal stem cells are derived from tissues from human being such as at least one of bone marrow tissue, adipose tissue, muscle tissue, reproductive tissue, skin tissue, bone tissue, and dental tissue, wherein the reproductive tissue comprises menstrual blood, amniotic membrane, amniotic fluid, or umbilical cord tissue; or prepared by separation or induction of human cells.

7. The use according to claim 1, wherein the mesenchymal stem cell is a homogeneous composition or a mixed cell cluster comprising the mesenchymal stem cells.

8. The use according to claim 1, wherein the drug for repairing lung damage comprises more than 95%, preferably more than about 98% by mass of the mesenchymal stem cells.

9. The use according to claim 1, wherein the drug for repairing lung damage is in a form of injection formulation.

10. The use according to claim 9, wherein the drug for repairing lung damage is a suspension of mesenchymal stem cells in physiological saline at a cell concentration of 0.5×10⁵ cells/mL to 5.0×10⁵ cells/mL.

11. A method for repairing lung damage, comprising administering a mesenchymal stem cell to a subject with lung damage.

12. The method according to claim 11, wherein the subject comprises a mammal who suffers from damage to lung in function and/or tissue.

13. The method according to claim 12, wherein the damage to lung in function and/or tissue comprises lung damage caused by a respiratory infection.

14. The method according to claim 12, wherein the lung damage comprises lung fibrosis.

15. The method according to claim 13, wherein the respiratory infection comprises viral pneumonia, bacterial pneumonia or fungal infection.

16. The method according to claim 15, wherein the viral pneumonia is a severe or critical type of pneumonia caused by any one or more of coronavirus SARS-CoV-2, SARS-Cov or MERS-Cov.

17. The method according to claim 11, wherein the mesenchymal stem cells are derived from tissues from human being such as bone marrow tissue, adipose tissue, muscle tissue, reproductive tissue, skin tissue, bone tissue, and dental tissue, wherein the reproductive tissue comprises menstrual blood, amniotic membrane, amniotic fluid, or umbilical cord tissue; or prepared by separation or induction of human cells.

18. The method according to claim 17, wherein the mesenchymal stem cell is a homogeneous composition or a mixed cell cluster comprising the mesenchymal stem cells.
